(19) Europäisches Patentamt
European Patent Office
Office européen des brevets

(11) Veröffentlichungsnummer: **0 212 400 B1**

(12) # EUROPÄISCHE PATENTSCHRIFT

(45) Veröffentlichungstag der Patentschrift: **02.01.92**

(51) Int. Cl.$^5$: **C07H 15/10**

(21) Anmeldenummer: **86110694.6**

(22) Anmeldetag: **02.08.86**

(54) **Neues Verfahren zur Herstellung von Sphingosinderivaten.**

(30) Priorität: **13.08.85 CH 3472/85**
**07.03.86 CH 938/86**

(43) Veröffentlichungstag der Anmeldung:
**04.03.87 Patentblatt 87/10**

(45) Bekanntmachung des Hinweises auf die Patenterteilung:
**02.01.92 Patentblatt 92/01**

(84) Benannte Vertragsstaaten:
**AT BE CH DE FR GB IT LI LU NL SE**

(56) Entgegenhaltungen:

**TETRAHEDRON LETTERS, Band 27, Nr. 4, 1986, Seiten 481-484, Pergamon Press Ltd, GB; R.R. SCHMIDT et al.: "Synthesis of D-Erythro-sphingosines"**

**ANGEW. CHEM. INT. ED. ENGL., Band 24, Nr. 1, 1985, Seiten 65-66, VCH Verlagsgesellschaft mbH, Weinheim, DE; R.R. SCHMIDT et al.: "Short synthesis of cerebrosides"**

**TETRAHEDRON LETTERS, Band 24, Nr. 49, 1983, Seiten 5491-5494, Pergamon Press Ltd, GB; B. BERNET et al.: "Enantioselective synthesis of D-erythro-sphingosine"**

**ANGEW. CHEM. INT. ED. ENGL., Band 21, Nr. 3, 1982, Seiten 210-211, Verlag Chemie GmbH, Weinheim, DE; R.R. SCHMIDT et al.: "Diastereoselective synthesis of D,L-sphingosine"**

(73) Patentinhaber: **Solco Basel AG**
**Gellertstrasse 18**
**CH-4052 Basel(CH)**

(72) Erfinder: **Schmidt, Richard R., Prof. Dr.**
**Grossherzog-Friedrich-Strasse 11**
**W-7750 Konstanz 16(DE)**
Erfinder: **Zimmermann, Peter**
**Ruhesteinweg 14**
**W-7730 Villingen(DE)**

(74) Vertreter: **Frossard, Michel, Dr. et al**
**A. Braun, Braun, Héritier, Eschmann AG, Patentanwälte Holbeinstrasse 36-38**
**CH-4051 Basel(CH)**

EP 0 212 400 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der europäischen Patentanmeldung Nr. 84114415.7 (Veröffentlichungs-Nr. 146 810) sind neue Sphingosinderivate der Formeln:

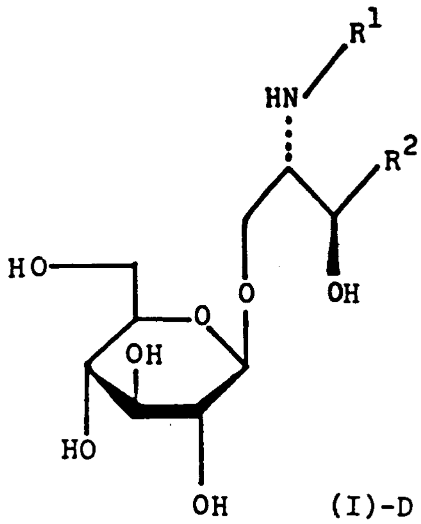

(I)-D

und

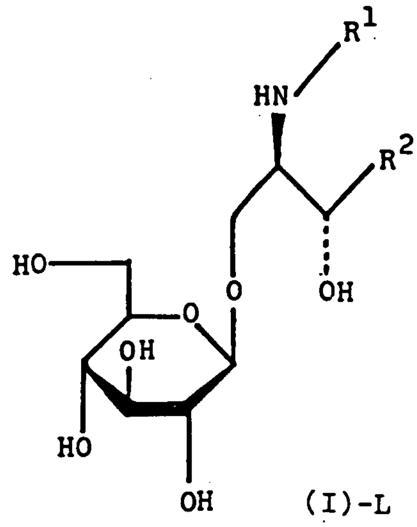

(I)-L

und Verfahren zu ihrer Herstellung.

In den obigen Formeln bedeuten $R^1$ den Acylrest einer Fettsäure mit 14 bis 24 Kohlenstoffatomen oder die entsprechenden Acylreste mit einer Hydroxylgruppe in $\alpha$-Stellung oder mit einer oder zwei Doppelbindungen in cis-Konfiguration und $R^2$ den Pentadecanyl- oder Heptadecanylrest oder die entsprechenden $C_{15}$- und $C_{17}$-Reste mit einer, zwei oder drei Doppelbindungen, von welchen jeweils eine in 1,2-Stellung sitzt und trans-Konfiguration aufweist, die andere oder anderen, wenn vorhanden, cis-Konfiguration aufweisen.

Diese Verbindungen besitzen die erythro-Konfiguration und entsprechen den bereits bekannten neutralen Glykosphingolipiden. Sie zeichnen sich durch wundheilungsfördernde bzw. zell- und geweberegenerierende Eigenschaften aus und eignen sich für eine therapeutische Anwendung bei Wunden jeglicher Genese, insbesondere bei schlecht oder langsam heilenden Wunden oder Ulzerationen. In der Tat führen sie, insbesondere bei topischer Anwendung auf Wunden, zur Bildung von gesundem, gut durchblutetem neuem Gewebe ohne störende Narben. Bevorzugt werden die Sphingosinderivate der Formel (I)-D wegen ihrer höheren therapeutischen Wirksamkeit.

Die Herstellung der oben erwähnten Verbin-dungen geht von entsprechenden Ceramiden der Formeln:

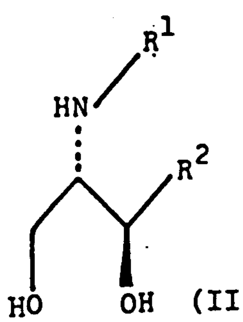

(II)-D

und/oder

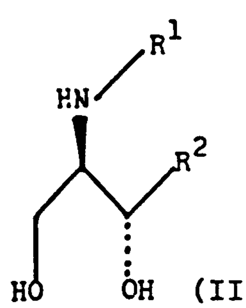

(II)-L

aus. Die Ceramide ihrerseits können aus den $C_{18}$- oder $C_{20}$-Sphingosinen durch N-Acylierung mittels einer Fettsäure der Formel $R^1$-OH hergestellt werden. Je nachdem, ob als Ausgangsprodukt ein optisch aktives oder ein racemisches Sphingosin eingesetzt wird, erhält man die Verbindungen der Formel (I)-D oder (I)-L in optisch einheitlicher Form oder aber ein Gemisch der Diastereomeren (I)-D und (I)-L; im letzteren Fall muss auf einer bestimmten Verfahrensstufe eine Trennung der Diastereomeren vorgenommen werden.

Die racemischen Sphingosine können neuerdings mit guter Ausbeute aus Glycin durch eine einfache

Synthese von R.R. Schmidt und R. Kläger [Angew. Chem. 94, 215 - 216 (1982); Angew. Chem. Int. Ed. Engl. 21, 210 - 211 (1982); Angew. Chem. Suppl. 1982, 393 - 397] erhalten werden. Obschon das oben besprochene Herstellungsverfahren die Sphingosinderivate der Formel (I)-D oder (I)-L ebenfalls mit befriedigender Ausbeute liefert, wäre einem Verfahren der Vorzug zu geben, welches ohne Auftrennung von Diastereomeren auskommt - zumal wenn man bedenkt, dass die wirksameren Verbindungen der D-Reihe angehören.

Andererseits sind verschiedene Synthesen bekannt, welche als Ausgangsprodukt eine ad hoc ausgewählte chirale Verbindung benutzen und somit ohne Auftrennung von Diastereomeren zu den optisch aktiven Sphingosinen von erythro-Konfiguration und der D-Reihe, mithin zu den in der Natur vorkommenden Sphingosinen führen.

Die etwas ältere Synthese von E.J. Reist und P.H. Christie [J. Org. Chem. 35, 3521 und 4127 (1970)], ausgehend von D-Glucose, und jene von H. Newman [J. Am. Chem. Soc. 95, 4098 (1973)] sowie von P. Tkaczuk und E.R. Thornton [J. Org. Chem. 46, 4393 (1981)], beide ausgehend von L-Serin, beinhalten jeweils eine Reaktionsstufe mit geringer Ausbeute, nämlich die Herstellung der 3-Amino-3-desoxy-di-(O-isopropyliden)-$\alpha$-D-allofuranose bzw. die Additionsreaktion von trans-Vinylalan und einem von L-Serin abgeleiteten Aldehyd.

Eine neuere Synthese von B. Bernet und A. Vasella [Tetrahedron Letters 24, 5491 - 5494 (1983)] ergibt das D-erythro-$C_{18}$-Sphingosin nach 6 Reaktionsstufen mit einer Gesamtausbeute von 33%. Sie geht allerdings von dem nicht unmittelbar erhältlichen Pentadecyn aus, dessen Herstellung sich auf die Anzahl Stufen und die Gesamtausbeute abträglich auswirkt.

Schliesslich sei noch die Synthese eines Ceramids durch K. Koike, Y. Nakahara und T. Ogawa [Glycoconjugate J. 1, 107 - 109 (1984)] erwähnt, welche von einem D-Glucosederivat ausgeht, 12 Reaktionsstufen umfasst und das Ceramid mit einer Ausbeute von etwa 20% liefert. Das Verfahren dürfte sich zur Herstellung der Sphingosine natürlicher Konfiguration anwenden lassen.

Bei der eingangs besprochenen Herstellung der Sphingosinderivate der Formel (I)-D war also bisher die an sich vorteilhaftere Verwendung der optisch aktiven D-Sphingosine als Ausgangsprodukte durch deren arbeitsmässig aufwendige und/oder ihre ausbeutemässig unbefriedigende Beschaffung beeinträchtigt.

Es wurde nun gefunden, dass man zu optisch einheitlichen Sphingosinderivaten der Formel (I) - siehe Formelblatt - nach einem neuen Verfahren gelangen kann, welches von dem im Handel erhältlichen D-Galactose ausgeht, insgesamt 9 bzw. 12 Stufen umfasst und die gewünschten Verbindungen mit einer befriedigenden Gesamtausbeute ergibt.

In der Formel (I) bedeutet $R^1$ dieselben Acylreste wie oben bei der Besprechung der Formeln (I)-D und (I)-L angegeben wird, während $R^3$ einen aliphatischen Rest mit 13 bis 19 Kohlenstoffatomen, wovon mindestens 13 in gerader Kette und gegebenenfalls höchstens 4 als seitliche Methylgruppen vorliegen, welcher Rest bis zu drei Doppelbindungen von cis- oder trans-Konfiguration oder bis zu drei Dreifachbindungen beinhalten kann, darstellt.

Das erfindungsgemässe Verfahren besteht darin, dass man D-Galactose mit einem niederen aliphatischen Keton oder einem aromatischen Aldehyd der Formel R-CO-R', in welcher R und R' je einen niederen Alkylrest bedeuten bzw. eines von R und R' das Wasserstoffatom und das andere einen aromatischen Rest bedeutet, zu einer in den 4- und 6-Stellungen geschützten D-Galactose der Formel (II) umsetzt, diese Verbindung mit einem vicinale Diole spaltenden Oxydationsmittel zur entsprechenden, in den 2- und 4-Stellungen geschützten D-Threose der Formel (III) aufspaltet, die geschützte D-Threose mit einem $R^3$-$CH_2$-Phosphonat oder einem $R^3$-$CH_2$-Triphenylphosphoniumhalogenid, in welchen $R^3$ die obige Bedeutung besitzt, in Gegenwart einer Base bzw. einer Base und eines Salzes zu eher Verbindung der Formel (IV) umsetzt, in dieser Verbindung die freie Hydroxylgruppe durch Aktivierung in eine Azidogruppe überführt, die erhaltene Azidoverbindung der Formel (V) von der Schutzgruppe der Hydroxylgruppen in den 1- und 3-Stellungen der aliphatischen Kette unter Bildung einer 2-Azido-1,3-dihydroxyverbindung der Formel (VI) befreit, letztere mit einem organischen Reagens, das mit einer primären Hydroxylgruppe selektiv zu reagieren vermag, unter Bildung einer Verbindung der Formel (VIII), in welcher R'' eine Hydroxylschutzgruppe bedeutet, umsetzt, in der Verbindung der Formel (VIII) die sekundäre Hydroxylgruppe mit einer Schutzgruppe R''' blockiert, von der erhaltenen Verbindung der Formel (IX) die Hydroxylschutzgruppe R'' unter Bildung einer Verbindung der Formel (X) abspaltet und entweder die zuvor erhaltene Verbindung der Formel (VI) oder aber die Verbindung der Formel (X) mit dem O-Trifluor- oder O-Trichlor-acetimidat oder dem 1-Halogenderivat einer D-Glucose, deren Hydroxylgruppen in den 2-, 3-, 4- und 6-Stellungen durch Acylreste Ac geschützt sind, zu einer Verbindung der Formel (VII) bzw. (XI) glykosidiert, von der erhaltenen Verbindung die Acylgruppen Ac bzw. die Acylgruppen Ac und die Schutzgruppe R''' unter Bildung derselben Verbindung der Formel (XII) abspaltet, in dieser die Azidogruppe in eine primäre Aminogruppe überführt und die erhaltene Verbindung der Formel (XIII) einer N-Acylierung mit einer Fettsäure der Formel

$R^1$-OH unterwirft.

Im folgenden wird die Erfindung ausführlicher beschrieben.

Die organische Carbonsäure $R^1$-OH, von welcher die Acylgruppe $R^1$ in den Sphingosinderivaten der Formel (I) abgeleitet ist, ist beispielsweise die Myristinsäure $C_{14}H_{28}O_2$, die Palmitinsäure $C_{16}H_{32}O_2$, die Stearinsäure $C_{18}H_{36}O_2$, die Oelsäure $C_{18}H_{34}O_2$, die Linolsäure $C_{18}H_{32}O_2$, die Arachinsäure $C_{20}H_{40}O_2$, die Behensäure $C_{22}H_{44}O_2$ und - an der oberen Grenze der für $R^1$ angegebenen Bedeutung - die Tetracosansäure (Lignocerinsäure) $C_{24}H_{48}O_2$, die cis-15-Tetracosensäure (Nervonsäure) $C_{24}H_{46}O_2$, die 2-Hydroxytetracosansäure (Cerebronsäure) $C_{24}H_{48}O_3$, die 2-Hydroxy-15-tetracosensäure (Hydroxynervonsäure) $C_{24}H_{46}O_3$ oder die mit letzteren isomere 2-Hydroxy-17-tetracosensäure.

Der aliphatische Rest $R^3$ kann eine unverzweigte Kette sein oder eine, zwei, drei oder vier Methylgruppen als Substituenten tragen. Ferner kann die Kette gesättigt oder ungesättigt sein; im letzteren Fall weist sie eine bis drei Doppelbindungen bzw. eine bis drei Dreifachbindungen auf. Die Doppelbindungen haben die cis- oder die trans-Konfiguration. Bevorzugte aliphatische Reste $R^3$ sind solche mit ungerader Anzahl Kohlenstoffatome, insbesondere die $C_{13}$- und $C_{15}$-Reste.

Bei der ersten Stufe des Verfahrens kann man zum Schutz der Hydroxylgruppen in 4- und 6-Stellung der D-Galactose ein niederes aliphatisches Keton wie Aceton, Ethylmethylketon oder Diethylketon, oder ein Aldehyd der aromatischen Reihe wie Benzaldehyd oder ein am Phenylring substituiertes Benzaldehyd verwenden. Bevorzugt wird hiezu die Verwendung des Benzaldehyds. Als Kondensationsmittel für die Reaktion eignen sich im allgemeinen Lewissäuren, wie Zinkchlorid, Bortrifluorid, Aluminiumchlorid und Eisenchlorid, oder Brønstedsäuren, wie p-Toluolsulfonsäure. Die Ueberführung der D-Galactose in die 4,6-O-Benzyliden-D-galactose kann z.B. nach der Methode von E.G. Gros und V. Deulofeu [J. Org. Chem. 29, 3647-3654 (1964)] durch/geführt werden, die Umsetzung von D-Galactose mit Aceton zur 4,6-O-Isopropyliden-D-galactose kann nach der Methode von J. Gelas und D. Horton [Carbohydr. Res. 71, 103-121 (1979)] vorgenommen werden.

Das in der zweiten Verfahrensstufe verwendete Oxydationsmittel kann ein Alkalimetallperjodat, z.B. das Lithium-, Natrium- oder Kaliumsalz,oder Bleitetraacetat sein; vorzugsweise wird Natriumperjodat verwendet. Die Oxydation wird mit Vorteil bei einem pH-Wert um 7 bis 8, z.B. in einer entsprechenden Pufferlösung, und bei Raumtemperatur durchgeführt.

Die Wittig-Reaktion gemäss der dritten Verfahrensstufe wird in der Regel in einer Inertgasatmosphäre, z.B. unter Stickstoff, bei tiefen Temperaturen, z.B. bei -10 bis -20 °C, und bei Verwendung eines $R^3$-$CH_2$-Phosphoniumhalogenids in Anwesenheit eines Salzes, z.B. Lithiumbromid, Natriumchlorid oder Kaliumbromid, durchgeführt. Als Base eigenen sich unter anderem organische Lithiumverbindungen, insbesondere Phenyllithium oder Lithiummethylat, ferner Natriumamid, Natriummethylat und Natriumcarbonat. Als Lösungsmittel kann man aromatische Kohlenwasserstoffe wie Benzol, Toluol oder Xylol, oder Ether wie Diethylether, Tetrahydrofuran oder Dioxan verwenden; das Lösungsmittel soll wasserfrei sein.

Die Ueberführung der freien Hydroxylgruppe in eine Azidogruppe durch Aktivierung kann mit Vorteil durch 0-Sulfonierung der Verbindung (IV) und anschliessende Umsetzung des gebildeten 0-Sulfonylderivates, z.B. des Methansulfonyl-, Trifluormethansulfonyl- oder p-Toluolsulfonylderivates,mit einem Alkalimetallazid durchgeführt werden; dabei erfolgt eine Inversion der Konfiguration an $C_2$ der D-Threose. Die O-Sulfonierung kann nach den in "Ullmanns Encyklopädie der technischen Chemie", 4. Auflage, Band 11, Seiten 91 und folgende, Verlag Chemie GmbH, Weinheim BRD (1976), beschriebenen Methoden durchgeführt werden. Man verwendet in der Regel ein Säurehalogenid oder ein Säureanhydrid einer niederen aliphatischen Sulfonsäure oder einer monocyclischen aromatischen Sulfonsäure, beispielsweise Methansulfonylchlorid, p-Toluolsulfonylchlorid, Methansulfonsäureanhydrid oder Trifluormethansulfonsäureanhydrid. Die 0-Sulfonierung wird vorzugsweise in Gegenwart einer Base durchgeführt. Da wasserfreie Reaktionsbedingungen eingehalten und ein organisches Lösungsmittel wie Benzol, Toluol, Tetrahydrofuran, Diethylether oder Dichlormethan verwendet werden sollen, eignen sich als Base insbesondere tertiäre organische Basen wie Triethylamin, Dimethylanilin, Pyridin, Collidin, Lutidin und dergleichen. Die nachfolgende Umsetzung mit dem Alkalimetallazid, z.B. Lithium-, Natrium- oder Kaliumazid, wird mit Vorteil ohne Reinigung des O-Sulfonylderivates durchgeführt. Beide Reaktionen werden vorzugsweise in einer Inertgasatmosphäre, z.B. unter Stickstoff, und bei tiefen Temperaturen oder Raumtemperatur durchgeführt.

In der fünften Verfahrensstufe kann die Abspaltung der Schutzgruppe von der Verbindung (V) durch saure Hydrolyse erfolgen. Beispielsweise löst man die Verbindung in einem organischen Lösungsmittel wie Dichlormethan oder Dimethylformanid und lässt dann eine kleine Menge konzentrierte Salzsäure und Wasser eine Zeit lang, vorzugsweise bei Raumtemperatur, einwirken.

Nun kann die Verbindung (VI) der Glykosidierung unter Bildung einer Verbindung (VII) direkt unterworfen werden oder aber über die Zwischenprodukte (VIII), (IX) und (X) in eine Verbindung (XI) umgewandelt werden, welche erst der Glykosidierung unterworfen wird. Diese zweite Verfahrensvariante umfasst zwar

drei Reaktionsstufen mehr, sie ergibt jedoch eine höhere Gesamtausbeute und eignet sich deshalb besonders gut für eine Produktion in industriellem Massstab. Sie wird im folgenden näher erläutert.

Der Schutz der primären Hydroxylgruppe der 2-Azido-1,3-dihydroxyverbindung (VI) soll mit Reagenzien vorgenommen werden, welche in Gegenwart einer primären und einer sekundären Hydroxylgruppe selektiv mit der ersteren reagieren. Es eignen sich als Schutzgruppe R" insbesondere solche, die eine grosse räumliche Beanspruchung aufweisen wie die tert.Butyl-, Triphenylmethyl- (Trityl-), Trichloracetyl-, Trimethylsilyl-, tert.Butyldimethylsilyl- oder tert.Butyldiphenylsilylgruppe. Bevorzugt werden die Triphenylmethyl-, Monomethoxytriphenylmethyl-, tert.Butyldimethylsilyl- und tert.Butyldiphenylsilylgruppe.

Die Einführung der Schutzgruppe R" erfolgt nach den bekannten Methoden der organischen Chemie, entsprechend der Art der gewählten Schutzgruppe. Beispielsweise kann die Triphenylmethylgruppe durch Behandeln der Verbindung (VI) mit einem entsprechenden Halogenid wie das Triphenylchlormethan oder das Triphenylbrommethan eingeführt werden. Auch für die tert.Butyldimethylsilyl- und die tert.Butyldiphenylsilylgruppe kann das entsprechende Halogenid, vorzugsweise das Chlorid oder das Bromid, mit Vorteil benützt werden.

Hierauf wird die in 1-Stellung geschützte Verbindung der Formel (VIII) an der Hydroxylgruppe in 3-Stellung durch eine Schutzgruppe R"' geschützt, z.B. durch Veresterung mit einer organischen Carbonsäure Ac'OH oder einem reaktionsfähigen funktionellen Derivat derselben. Es eignen sich dazu vor allem einfache, aliphatische Carbonsäuren und aromatische, insbesondere monocyclische aromatische Carbonsäuren; bevorzugt wird die Verwendung der Benzoesäure, einer substituierten Benzoesäure oder der Pivalinsäure.

Die Veresterung mit der Carbonsäure Ac'OH kann nach den in "Ullmanns Encyklopädie der technischen Chemie", 4. Auflage, Band 11, Seiten 91 und folg., Verlag Chemie GmbH, Weinheim BRD (1976), beschriebenen Methoden durchgeführt werden. Sie erfolgt mit Vorteil unter Benutzung eines Carbonsäurehalogenids in Gegenwart einer tertiären organischen Base wie Triethylamin, Pyridin oder Dimethylanilin, in einem wasserfreien organischen Lösungsmittel wie Benzol, Toluol, Tetrahydrofuran, Diethylether oder Dichlormethan.

Die Schutzgruppe R" der Hydroxylgruppe in 1-Stellung der Verbindung der Formel (IX) kann durch saure Hydrolyse (Triphenylmethylschutzgruppen, Silylschutzgruppen) oder durch Behandlung mit Bortrifluorid-etherat (Triphenylmethylgruppen) abgespaltet werden. Erhalten wird die Verbindung der Formel (X), in welcher die Hydroxylgruppe in 3-Stellung weiterhin durch die Schutzgruppe R"' blockiert, die primäre Hydroxylgruppe in 1-Stellung aber wieder frei ist.

Die Umsetzung der Verbindung (IX) oder aber jene der Verbindung (VI) mit dem O-Trichlor- oder O-Trifluor-acetimidat einer D-Glucose, deren Hydroxylgruppen ausser jener an der 1-Stellung durch Acylreste Ac geschützt sind, wird mit Vorteil durch eine Lewis-Säure wie Bortrifluorid-etherat oder Trifluormethansulfonsäuretrimethylsilylester katalysiert. Sie wird im allgemeinen in einem wasserfreien osganischen Lösungsmittel wie ein Kohlenwasserstoff (Hexan) oder ein halogenierter Kohlenwasserstoff (Dichlormethan)-durchgeführt. Als Acylreste zum Schutz der Hydroxylgruppen in den 2-, 3-, 4- und 6-Stellungen der D-Glucose werden vorzugsweise niedere aliphatische Acylgruppen wie die Acetyl-, Propionyl-, Pivaloyl-, Trifluoracetyl- oder Methansulfonylgruppe verwendet. Einzelheiten über die Herstellung des Reagens können der Abhandlung von R.R. Schmidt und M. Stumpp (Liebigs Ann. Chem. 1983, 1249-1256) und R.R. Schmidt, J. Michel und M. Roos (Liebigs Ann. Chem. 1984, 1343-1357) entnommen werden.

Die entsprechende Umsetzung mit dem 1-Halogenderivat der O-tetraacylierten D-Glucose, beispielsweise mit dem O-Acetyl-α-D-glucopyranosylchlorid oder -bromid (letzteres auch α-D-O-Acetobromglucose genannt), wird in der Regel in Gegenwart einer Schwermetallverbindung wie Silberoxid, eines Schwermetallsalzes, wie Silbercarbonat oder Quecksilbercyanid, oder einer organischen Base, welche als säurebindende Mittel fungieren, durchgeführt (Ullmanns Encyklopädie der technischen Chemie, 4. Auflage, Band 24, Seite 757, Verlag Chemie GmbH, Weinheim BRD 1983).

Die Abspaltung der Acylreste Ac und der Schutzgruppe R"' aus der Verbindung (VII) bzw. (XI) wird im allgemeinen durch Basen katalysiert; besonders zweckmässig dafür ist die Verwendung von Natriummethanolat in wasserfreiem Methanol bei Raumtemperatur.

In der vorletzten Verfahrensstufe wird die Ueberführung der Azidogruppe in die primäre Aminogruppe am besten durch Behandlung der Verbindung (XII) mit Schwefelwasserstoff bei Raumtemperatur bewerkstelligt. Hierfür wird die Verbindung beispielsweise in einem Gemisch (1:1) von Wasser und Pyridin aufgelöst. Dieselbe Ueberführung kann auch durch Hydrierung mit Natriumborhydrid oder einem anderen Reduktionsmittel, wie z.B. Natriumcyanoborhydrid, durchgeführt werden.

Die N-Acylierung der Verbindung (XIII) mit der organischen Carbonsäure der Formel $R^1$-OH (letzte Verfahrensstufe) kann nach der Methode von D. Shapiro und Mitarbeiter [J. Am. Chem. Soc. 86, 4472 (1964)] durchgeführt werden. Im allgemeinen wird man die Carbonsäure selbst in Gegenwart eines

wasserabspaltenden Mittels, wie Dicyclohexylcarbodiimid in Dichlormethan, oder ein funktionelles reaktionsfähiges Derivat der Carbonsäure,wie einen aktivierten Ester oder ein Halogenid in Gegenwart einer anorganischen Base wie Natriumacetat oder einer tertiären organischen Base, einsetzen. Die N-Acylierung wird mit Vorteil bei Raumtemperatur durchgeführt.

Die Isolierung und Reinigung der bei jeder Verfahrensstufe anfallenden Verbindungen erfolgt nach den üblichen Methoden der organischen Chemie.

Die folgenden Beispiele veranschaulichen bevorzugte Ausführungsformen der Erfindung.

$^1$H-NMR-Spektren wurden mit dem 250 MHz-Gerät WM 250 Cryospec der Firma Bruker, Spectrospin, Industriestrasse 26, CH-8117 Fällanden/Zürich, gemessen. Die Verschiebungen sind auf Tetramethylsilan (TMS) als internen Standard bezogen und in ppm angegeben.

Die angegebenen Schmelzpunkte wurden auf einem Kupferblock bestimmt und sind nicht korrigiert.

Zur analytischen Dünnschichtchromatographie (DC) wurden Kieselgelplatten der Firma E. Merck AG, Darmstadt (BRD), verwendet. Die Dünnschichtchromatogramme wurden, sofern die Substanzen nicht UV-aktiv waren, mit 15% Schwefelsäure besprüht und bei 120 °C entwickelt.

Präparative Säulenchromatographien wurden mit Kieselgel 60(0,062-0,200 mm) der Firma Merck durchgeführt. Für die Mitteldruckchromatographie wurden Fertigsäulen nach D. Flockerzi, Diplomarbeit, Universität Stuttgartt/BRD (1978), mit Kieselgel "LiChroprep Si 60,15-25" verwendet.

Die Ausbeuten wurden auf der Reinigungsstufe angegeben, auf der NMR-spektroskopisch und mittels Dünnschichtchromatographie keine Verunreinigungen nachzuweisen waren.

Bei den Lösungsmittelgemischen bedeutet die Angabe in Klammern Volumenteile.

## Beispiel 1

### 2S,3R-2-Hexadecanoylamino-3-hydroxy-1-(β-D-glucopyranosyloxy)-4-trans-eicosen

a) 4,6-O-Benzyliden-D-galactose

Siehe J. Org. Chem. 29, 3647-3654 (1964).

b) 2,4-O-Benzyliden-D-threose (1)

30 g (0,111 Mol) 4,6-O-Benzyliden-D-galactose werden in ca. 1200 ml Phosphatpuffer von pH 7,6 gelöst. 55 g (0,257 Mol) Natriumperjodat werden unter kräftigem Rühren hinzugegeben. Der pH-Wert wird durch Zutropfen von 2 n Natronlauge auf ca. 7 bis 8 gehalten. Man lässt 1,5 Stunden lang bei Raumtemperatur rühren. Danach wird im Wasserstrahlvakuum bis zur Trockene eingeengt. Der feste Rückstand wird viermal mit je 250 ml Essigester extrahiert. Der Extrakt wird filtriert, über Magnesiumsulfat getrocknet und eingeengt. Ausbeute: 20 g (85%), $R_F$ = 0,64 in Toluol/Ethanol (3:1).

c) 2R,3R-1,3-O-Benzyliden-2-hydroxy-4-trans-eicosen (2)

70 g (0,12 Mol) Hexadecyltriphenylphosphoniumbromid werden unter Stickstoff in ca. 1 Liter wasserfreiem, stickstoffgesättigtem Toluol suspendiert. Phenyllithium, welches aus 6,5 g (0,94 Mol) Lithium und 74 g (0,47 Mol) Brombenzol in ca. 200 ml wasserfreiem Ether dargestellt wurde, wird ohne weitere Reinigung hinzugetropft. Gleichzeitig wird das Gemisch auf -15 °C abgekühlt. Danach werden 20 g (0,096 Mol) Verbindung (1) in ca. 150 ml wasserfreiem Tetrahydrofuran unter Stickstoff während 20 Minuten zugetropft. Nach weiteren 20 Minuten werden zunächst 150 ml Methanol und danach 250 ml Wasser hinzugegeben. Es wird kräftig gerührt. Die organische Phase wird nach Abtrennen der wässrigen Phase eingeengt. Zur Reinigung wird über Kieselgel mit Petrolether/Essigester (9:1) chromatographiert. Ausbeute: 27 g (68%), $R_F$ = 0,21 in Petrolether/Essigester (9:1).

d) 2S,3R-2-Azido-1,3-O-benzyliden-4-trans-eicosen (3)

10 g (0,025 Mol) Verbindung (2) werden in ca. 70 ml wasserfreiem Dichlormethan, welches 5 ml wasserfreies Pyridin enthält, gelöst. Es wird unter Stickstoff auf -15 °C gekühlt. 8,12 g (0,029 Mol) Trifluormethansulfonsäureanhydrid werden langsam zugetropft. Nach 15 Minuten wird über Kieselgel filtriert und mit Dichlormethan/Petrolether (1:1) eluiert. Die Vorlage wird ständig mit Stickstoff gespült. Es wird eingeengt, und das zurückbleibende Oel wird in 50 ml wasserfreiem Dimethylformamid aufgenommen. Unter Stickstoff werden 7,5 g (0,1 Mol) Natriumazid hinzugegeben. Man lässt 2 Stunden bei Raumtemperatur rühren. Danach wird mit ca. 350 ml Dichlormethan verdünnt, filtriert und im Wasserstrahlvakuum eingeengt. Zur Reinigung wird über Kieselgel mit Petrolether/Essigester (9:1) chromatographiert. Ausbeute: 8 g (75%), $R_F$ = 0,8 in Petrolether/Essigester (9:1).

e) 2S,3R-2-Azido-1,3-dihydroxy-4-trans-eicosen (4)

8 g (0,018 Mol) Verbindung (3) werden in 100 ml Dichlormethan gelöst. Man gibt 5 ml konzentrierte Salzsäure und 3 ml Wasser hinzu und lässt bei Raumtemperatur 12 Stunden lang kräftig rühren. Danach wird mit wässriger Natriumhydrogencarbonatlösung ausgeschüttelt. Die organische Phase wird abgetrennt, über Natriumsulfat getrocknet und eingeengt. Zur Reinigung wird über Kieselgel mit Dichlormethan/Methanol (95:5) chromatographiert. Ausbeute: 4,32 g (68%), $R_F$ = 0,46 in Dichlormethan/Methanol (95:5), Schmelzpunkt: 56-57° C.

| | | | | |
|---|---|---|---|---|
| Elementaranalyse: | ber. | C 67,95 | H 11,11 | N 11,88 |
| | gef. | 67,62 | 11,12 | 11,85 |

$^1$H-NMR (250 MHz, $CDCl_3$ in ppm) Verbindung (4):5,83 (m, 1H, $-CH_2-\underline{CH}=C$); 5,55 (dd, 1H, $-CH_2-CH=CH-$, J = 15,5 Hz, J = 6,5 Hz); 4,25 (m, 1H, $-CH-N_3$); 3,8 (m, 2H, $-\underline{CH_2}-OH$, CH-OH); 3,52 (m, 1H, $-CH_2-\underline{OH}$); 2,05 (m, 4H, OH, $C=CH-CH_2$); 1,45-1,18 (m, 26H, aliphat.); 0,88 (t, 3H, $CH_3$).

f) 2S,3R-2-Azido-3-hydroxy-1-(2,3,4,6-tetra-O-acetyl-β-D-glucopyranosyloxy)-4-trans-eicosen (6)

0,5 g (1,41 mMol) Verbindung (4) werden in 50 ml wasserfreiem Hexan gelöst. Es werden 0,1 ml 0,5 M Bortrifluorid-etherat in Dichlormethan und eine Spatelspitze Molekularsieb 4 Å zugesetzt. 0,7 g (1,41 mMol) O-(2,3,4,6-Tetra-O-acetyl-α-D-glucopyranosyl)-trichloracetimidat werden in 3 ml wasserfreiem Toluol gelöst und langsam zugetropft. Nach 4 Stunden wird mit 30 ml gesättigter Natriumhydrogencarbonatlösung gewaschen. Die wässrige Phase wird dreimal mit je 30 ml Dichlormethan ausgeschüttelt. Die organischen Phasen werden über Natriumsulfat getrocknet und eingeengt. Zur Reinigung wird über Kieselgel mit Dichlormethan/Methanol (97,5:2,5) chromatographiert. Ausbeute: 0,385 g (40%), $R_F$ = 0,7 in Dichlormethan/Methanol (95:5).

$^1$H-NMR (250 MHz, $CDCl_3$ in ppm) Verbindung (6):5,78 (m, 1H, $-CH_2-\underline{CH}=C$); 5,5 (dd, 1H, $-CH_2-CH=CH-$, J = 15,5 Hz, J = 7,3 Hz); 5,3-4,98 (m, 3H, H-2, H-3, H-4); 4.58 (d, 1H, H-1, J = 7,6 Hz); 4,35-4,13 (m, 3H, H-6, H-6; $-CH-N_3$); 4,05 (dd, 1H, $-CH_2-O-$); 3,73 (m, 2H, H-5, $-CH_2-O$); 3,47 (m, 1H, >CH-OH); 2,24 (d, 1H, OH, J = 4,8 Hz); 2,16-1,94 (m, 14H, Acetyl, $C=CH-\underline{CH_2}$); 1,45-1,15 (m, 26H, aliphat.); 0,88 (t, 3H, $-CH_3$).

g) 2S,3R-2-Azido-3-hydroxy-1-(β-D-glucopyranosyloxy)-4-trans-eicosen (7)

0,4 g (0,585 mMol) Verbindung (6) werden in 30 ml wasserfreiem Methanol gelöst. Es werden 0,2 ml einer 1 M Lösung von Natriummethylat in Methanol zugegeben. Man lässt eine Stunde bei Raumtemperatur rühren. Danach wird mit dem Ionenaustauscher Amberlit IR 120 ($H^{\oplus}$-Form) neutralisiert. Es wird vom Ionenaustauscher abfiltriert, eingeengt und über Kieselgel mit Chloroform/Methanol (9:1) chromatographiert. Ausbeute: 0,26 g (86%), $R_F$ = 0,22 in Chloroform/Methanol (9:1).

$^1$H-NMR (250 MHz, DMSO-$d_6$ in ppm) Verbindung (7):4,10 (d, 1H, H-1, J = 7,6 Hz).

h) 2S,3R-2-Amino-3-hydroxy-1-(β-D-glucopyranosyloxy)-4-trans-eicosen (8)

0,26 g (0,5 mMol) Verbindung (7) werden in einem Gemisch aus 4 ml Pyridin und 4 ml Wasser gelöst. Die Lösung wird mit Schwefelwasserstoff gesättigt. Man lässt 24 Stunden bei Raumtemperatur rühren. Es wird bis zur Trokkene eingeengt und über Kieselgel zuerst mit Chloroform/Methanol (6:4), dann mit Chloroform/Methanol/Wasser (5:4:1) chromatographiert. Ausbeute: 0,234 g (96%), $R_F$ = 0,65 in Chloroform/Methanol/Wasser (5:4:1).

$^1$H-NMR (250 MHz, DMSO-$d_6$ in ppm) Verbindung (8):4,10 (d, 1H, H-1, J = 7,6 Hz).

i) 2S,3R-2-Hexadecanoylamino-3-hydroxy-1-(β-D-glucopyranosyloxy)-4-trans-eicosen (9)

0,23 g (0,47 mMol) Verbindung (8) werden in 5 ml Tetrahydrofuran gelöst. 5 ml einer 50% wässrigen Natriumacetatlösung werden hinzugegeben. Man versetzt das Gemisch bei Raumtemperatur unter kräftigem Rühren mit 0,19 g (0,7 mMol) Hexadecanoylchlorid. Nach ca. 2 Stunden wird die organische Phase abgetrennt. Die wässrige Phase wird dreimal mit je 2 ml Chloroform ausgeschüttelt. Die organischen Phasen werden über Natriumsulfat getrocknet und eingeengt. Zur Reinigung wird über Kieselgel mit Chloroform/Methanol (9:1) chromatographiert. Ausbeute: 0,3 g (90%), $R_F$ = 0,50 in Chloroform/Methanol (9:1).

$^1$H-NMR (250 MHz, DMSO-$d_6$ in ppm) Verbindung (9):7,5 (d, 1H, NH, J = 8,7 Hz); 5,52 (m, 1H, $-CH_2-CH=C$); 5,35 (dd, 1H, $C=CH-$, J = 15,2 Hz, J = 6,5 Hz); 5,03 (d, 1H, OH, J = 3,4 Hz); 4,92 (m, 3H, OH); 4,5 (t, 1H, OH, J = 4,9 Hz); 4,09 (d, 1H, H-1, J = 7,6 Hz); 4,0-3,55 (m, 4H); 3,45 (m, 2H); 3,15-2,9 (m, 4H); 2,1-1,88 (m, 4H); 1,45 (m, 2H); 1,22 (m, 54H, aliphat.); 0,85 (m, 6H, $CH_3$).

Anhang

Zur Bestätigung der der Verbindung der Formel (VI) zugeschriebenen Struktur ist die Verbindung (4) derselben Behandlung mit Schwefelwasserstoff unterworfen worden (siehe unten), wie im Abschnitt (h) des vorangegegangenen Beispiels beschrieben wird. Dabei ist in der Tat die Verbindung (5) erhalten worden,

deren physikalisch-chemische Eigenschaften mit jenen des aus natürlichen Quellen hergestellten erythro-D-$C_{18}$-Sphingosins vollends übereinstimmen.

2S,3R-2-Amino-1,3-dihydroxy-4-trans-eicosen (5)

0,25 g (0,7 mMol) Verbindung (4) werden in einem Gemisch aus 5 ml Pyridin und 2 ml Wasser gelöst. Die Lösung wird mit Schwefelwasserstoff gesättigt. Man lässt 48 Stunden bei Raumtemperatur rühren. Es wird bis zur Trockene eingeengt. Der Rückstand wird über Kieselgel zunächst mit Chloroform, dann mit Chloroform/Methanol (9:1) und zuletzt mit Chloroform/Methanol/Wasser (8:2:0,25) chromatographiert. Ausbeute: 0,215 g (95%), $R_F$ = 0,2 in Chloroform/Methanol (1:1), Schmelzpunkt: 70-72 °C.
$^1$H-NMR (250 MHz, $CDCl_3$ in ppm) Verbindung (5):5,78 (m, 1H, -$CH_2$-CH-C); 5,47 (dd, 1H, -$CH_2$-CH=CH-, J = 15,5 Hz, J = 7,3 Hz); 4,12 (dd, 1H, C=CH-CH-OH, J = 6,1 Hz); 3,7 (m, 2H, $CH_2$-OH); 2,93 (m, 1H, -CH-$NH_2$); 2,57 (m, 4H, $NH_2$, OH); 2,06 (m, 2H, C=CH-$CH_2$); 1,45-1,18 (m, 26 H, aliphat.); 0,88 (t, 3H, -$CH_3$).

## Beispiel 2

2S,3R-2-Hexadecanoylamino-3-hydroxy-1-(β-D-glucopyranosyloxy)-4-trans-octadecen

j) 2R,3R-1,3-O-Benzyliden-2-hydroxy-4-trans-octadecen (10)
70 g (0,13 Mol) Tetradecyltriphenylphosphoniumbromid werden unter Stickstoff in ca. 1 Liter wasserfreiem, stickstoffgesättigtem Toluol suspendiert. Phenyllithium, welches aus 6,5 g (0,94 Mol) Lithium und 74 g (0,47 Mol) Brombenzol in ca. 200 ml wasserfreiem Ether dargestellt wurde, wird ohne weitere Reinigung hinzugetropft. Gleichzeitig wird das Gemisch auf -15 °C abgekühlt. Danach werden 21,6 g (0,104 Mol) 2,4-O-Benzyliden-D-threose [siehe Beispiel 1, Verbindung (1)] in ca. 150 ml wasserfreiem Tetrahydrofuran unter Stickstoff während 20 Minuten zugetropft. Nach weiteren 20 Minuten werden zunächst 150 ml Methanol und danach 250 ml Wasser hinzugegeben. Es wird kräftig gerührt. Die organische Phase wird nach Abtrennen der wässrigen Phase eingeengt. Zur Reinigung wird über Kieselgel mit Petrolether/Essigester (9:1) chromatographiert. Ausbeute: 27 g (68%), $R_F$ = 0,21 in Petrolether/Essigester (9:1), Schmelzpunkt: 54 - 55 °C.
k) 2S,3R-2-Azido-1,3-O-benzyliden-4-trans-octadecen (11)
10 g (0,025 Mol) Verbindung (10) werden in ca. 70 ml wasserfreiem Dichlormethan, welches 5 ml wasserfreies Pyridin enthält, gelöst. Es wird unter Stickstoff auf -15 °C gekühlt. 8,7 g (0,31 Mol) Trifluormethansulfonsäureanhydrid werden langsam zugetropft. Nach 15 Minuten wird über Kieselgel filtriert und mit Dichlormethan/Petrolether (1:1) eluiert. Die Vorlage wird ständig mit Stickstoff gespült. Es wird eingeengt, und das zurückbleibende Oel wird in 50 ml wasserfreiem Dimethylformamid aufgenommen. Unter Stickstoff werden 7,5 (0,1 Mol) Natriumazid hinzugegeben. Man lässt 2 Stunden bei Raumtemperatur rühren. Danach wird mit ca. 350 ml Dichlormethan verdünnt, filtriert und im Wasserstrahlvakuum eingeengt. Zur Reinigung wird über Kieselgel mit Petrolether/Essigester (9:1) chromatographiert. Ausbeute: 7,8 g (75%), $R_F$ = 0,8 in Petrolether/Essigester (9:1).
l) 2S,3R-2-Azido-1,3-dihydroxy-4-trans-octadecen (12)
7 g (0,017 Mol) Verbindung (11) werden in 100 ml Dichlormethan gelöst. Man gibt 5 ml konzentrierte Salzsäure und 3 ml Wasser hinzu und lässt bei Raumtemperatur 12 Stunden lang kräftig rühren. Danach wird mit wässriger Natriumhydrogencarbonatlösung ausgeschüttelt. Die organische Phase wird abgetrennt, über Natriumsulfat getrocknet und eingeengt. Zur Reinigung wird über Kieselgel mit Dichlormethan/Methanol (95:5) chromatographiert. Ausbeute: 3,76 g (68%), $R_F$ = 0,46 in Dichlormethan/Methanol (95:5).
$^1$H-NMR (250 MHz, $CDCl_3$ in ppm): Verbindung (12): 5,83 (m, 1H, $CH_2$-CH=C): 5,55 (dd, 1H, -$CH_2$-CH=CH-, J = 15,5 Hz, J = 6,5 Hz); 4,25 (m, 1H, -CH-$N_3$); 3,8 (m, 2H, -$CH_2$-OH, >CH-OH); 3,52 (m, 1H, -$CH_2$-OH); 2,05 (m, 4H, OH, C=CH-$CH_2$); 1,45-1,18 (m, 22H, aliphat.); 0,88 (t, 3H, $CH_3$).
m) 2S,3R-2-Azido-3-hydroxy-1-O-triphenylmethyl-4-trans-octadecen (13)
4 g (12,3 mMol) Verbindung (12) werden in 45 ml eines Gemisches aus jeweils wasserfreiem Pyridin/Chloroform/Tetrahydrofuran (1:1:1) gelöst. 6 g (21,5 mMol) Tritylchlorid werden hinzugegeben. Die Mischung wird 48 Stunden bei Raumtemperatur gerührt. Danach engt man im Wasserstrahlvakuum ein. Der Rückstand wird in 200 ml Diethylether aufgenommen und mit 100 ml Wasser ausgeschüttelt. Die organische Phase wird über Magnesiumsulfat getrocknet und eingeengt. Zur Reinigung wird über Kieselgel mit Petrolether/Essigester (9:1) chromatographiert. Ausbeute: 6,3 g (90 %), $R_F$ = 0,39 in Petrolether/Essigester (9:1).

$^1$H-NMR (250 MHz, $CDCl_3$ in ppm) Verbindung (13): 7,55-7,15 (m, 15 H, aromat.); 5,75-5,58 (m, 1H,

$CH_2$-CH = C); 5,38-5,26 (dd, 1H, $CH_2$-CH = CH-, J = 15,5 Hz, J = 7,3 Hz); 4,20 (m, 1H, -CH-$N_3$); 3,53 (m, 1H, -CH-OH); 3,30 (d, 2H, O-$CH_2$-, J = 5,4 Hz); 2,03-1,188 (m, 3H, -OH, CH = CH-$CH_2$); 1,40-1,10 (m, 22H, aliphat.); 0,88 (t, 3H, $CH_3$).

n) 2S,3R-2-Azido-3-benzoyloxy-1-O-triphenylmethyl-4-trans-octadecen (14)

6,3 g (11,1 mMol)Verbindung (13) werden in 30 ml eines Gemisches aus jeweils wasserfreiem Toluol/Pyridin (4:1) gelöst. 3 g (21,3 mMol) Benzoylchlorid werden hinzugefügt. Man lässt 12 Stunden bei Raumtemperatur rühren. Danach wird auf ca. 200 ml Wasser gegossen und zweimal mit jeweils 100 ml Diethylether extrahiert. Die organische Phase wird über Magnesiumsulfat getrocknet und eingeengt. Zur Reinigung wird über Kieselgel mit Petrolether/Essigester (95:5) chromatographiert. Ausbeute: 6,7 g (90 %), $R_F$ = 0,60 in Petrolether/Essigester (9:1).

o) 2S,3R-2-Azido-3-benzoyloxy-1-hydroxy-4-trans-octadecen (15)

6,7 g (9,97 mMol) Verbindung (14) werden in einem Gemisch aus 30 ml wasserfreiem Toluol und 5 ml wasserfreiem Methanol gelöst. 10 ml 3 M Bortrifluorid-etherat in Dichlormethan werden hinzugegeben. Nach 5 Stunden giesst man auf 50 ml Wasser und trennt die organische Phase ab. Nach Trocknen über Magnesiumsulfat wird eingeengt und zunächst mit Petrolether/Essigester (9:1), dann mit Petrolether/Essigester (8:2) chromatrographiert. Ausbeute: 3,8 (90 %), $R_F$ = 0,13 in Petrolether/Essigester (9:1).

| | | | | |
|---|---|---|---|---|
| Elementaranalyse für $C_{25}H_{39}N_3O_3$ | ber.: | C 69,90 | H 9,14 | N 9,78 |
| (MG 429,56) | gef.: | 69,92 | 9,16 | 9,65 |

$^1$H-NMR (250 MHz, $CDCl_3$ in ppm) Verbindung (15); 8,14 (m, 2H, aromat.); 7,58 (m, 1H, aromat.); 7,47 (m, 2H, aromat.); 6,05-5,87 (m, 1H, $CH_2$-CH = C); 5,69-5,53 (m, 2H, $CH_2$-CH = CH-, CH-OBz); 2,15-1,95 (m, 3H, -OH, C = CH-$CH_2$); 1,47-1,13 (m, 22H, aliphat.); 0,86 (t, 3H, $CH_3$).

p) 2S,3R-2-Azido-3-benzoyloxy-1-(2,3,4,6-tetra-O-pivaloyl-β-D-glucopyranosyloxy)-4-trans-octadecen - (16)

2 g (4,6 mMol) Verbindung (15) und 4,6 g (7,0 mMol) 2,3,4,6-tetra-O-pivaloyl-α-D-glucopyranosyltrichloracetimidatwerden in 40 ml wasserfreiem Dichlormethan gelöst und 30 Minuten lang mit Molekularsieb 4 Å gerührt. Danach werden 0,2 ml 0,1 M Bortrifluorid-etherat in Dichlormethan zugegeben. Im Verlauf der Reaktion werden noch 2 ml 0,1 M Bortrifluorid-etherat in Portionen von jeweils 0,5 ml zugegeben. Nach 48 Stunden wird mit 200 ml Petrolether verdünnt und abfiltriert. Man schüttelt das Filtrat mit 50 ml wässriger Natriumhydrogencarbonatlösung aus, trocknet die organische Phase über Natriumsulfat und engt ein. Zur Reinigung wird über Kieselgel mit Toluol/Aceton (97,5:2,5) chromatographiert. Ausbeute 4 g (94 %), $R_F$ = 0,57 in Toluol/Aceton (97,5:2,5).

$^1$H-NMR (250 MHz, $CDCl_3$ in ppm) Verbindung (16): 8,05 (m, 2H, aromat.); 7,58 (m, 1H, aromat.); 7,45 (m, 2H, aromat.); 5,99-5,83 (m, 1H, $CH_2$-CH = C); 5,65-5,46 (m, 2H, $CH_2$-CH = CH, CH-OBz); 5,37-5,02 (m, 3H, H-2, H-3, H-4); 4,58 (d, 1H, H-1, 1 = 7,9 Hz); 4,25-3,58 (m, 6H, H-6, H-6', H-5, CH-$N_3$, $CH_2$-O); 2,06 (m, 2H, CH = CH-$CH_2$); 1,45-1,04 (m, 58H, Pivaloyl, aliphat.); 0,89 (t, 3H, $CH_3$).

q) 2S,3R-2-Azido-3-hydroxy-1-(β-D-glucopyranosyloxy)-4-trans-octadecen (17)

4 g (4,3 mMol) Verbindung (16) werden in 50 ml wasserfreiem Dichlormethan gelöst. 8 ml einer 0,05 M Natriummethylatlösung in wasserfreiem Methanol werden hinzugegeben. Man lässt drei Tage bei Raumtemperatur rühren. Danach wird mit Ionenaustauscher Amberlit JR 120 ($H^{\oplus}$-Form) neutralisiert. Es wird vom Ionenaustauscher abfiltriert, eingeengt und über Kieselgel mit Chloroform/Methanol (8,5:1,5) chromatographiert. Ausbeute: 1,65 g (78 %), $R_F$ = 0,20 in Chloroform/Methanol (9:1).

$^1$H-NMR (250 MHz, DMSO-$d_6$ in ppm) Verbindung (17): 4,10 (d, 1H, H-1, J = 7,6 Hz).

r) 2S,3R-2-Amino-3-hydroxy-1-(β-D-glucopyranosyloxy)-4-trans-octadecen (18)

1,65 g (3,4 mMol) Verbindung (17) werden in 50 ml eines Gemisches aus Pyridin/Wasser (1:1) gelöst. Die Lösung wird mit Schwefelwasserstoff gesättigt. Man lässt 24 Stunden bei Raumtemperatur rühren. Es wird bis zur Trockene eingeengt und über Kieselgel zuerst mit Chloroform/Methanol (9:1), dann mit Chloroform/Methanol/Wasser (5:4:1) chromatographiert. Ausbeute: 1,47 g (94 %), $R_F$ = 0,64 in Chloroform/Methanol/Wasser (5:4:1).

$^1$H-NMR (250 MHz, DMSO-$d_6$ in ppm) Verbindung (18): 4,10 (d, 1H, H-1, J = 7,6 Hz).

s) 2S,3R-2-Hexadecanoylamino-3-hydroxy-1-(β-D-glucopyranosyloxy)-4-trans-octadecen (19)

1,47 g (3,2 mMol) Verbindung (18) werden in 50 ml Tetrahydrofuran gelöst. 50 ml einer 50 % wässrigen Natriumacetatlösung werden hinzugegeben. Man versetzt das Gemisch bei Raumtemperatur unter kräftigem Rühren mit 0,87 g (3,2 mMol) Hexadecanoylchlorid. Nach ca. 2 Stunden wird die Mischung mit

350 ml Tetrahydrofuran verdünnt und die wässrige Phase abgetrennt. Die organische Phase wird zweimal mit jeweils 50 ml gesättigter Kochsalzlösung ausgeschüttelt und eingeengt. Der Rückstand wird am Hochvakuum getrocknet. Zur Reinigung wird über Kieselgel zuerst mit Chloroform, dann mit Chloroform/Methanol (9:1) chromatographiert. Ausbeute: 1,81 g (81 %), $R_F$ = 0,4 in Chloroform/Methanol (8,5:1,5).

$^1$H-NMR (250 MHz, DMSO-$d_6$ in ppm) Verbindung (19): 7,5 (d, 1H, NH, J = 8,7 Hz); 5,52 (m, 1H, -$CH_2$-CH=C); 5,35 (dd, 1H, $CH_2$-CH=CH-, J = 15,2 Hz, J = 6,5 Hz); 5,03 (d, 1H, OH, J = 3,4 Hz); 4,92 (m, 3H, OH); 4,5 (t, 1H, OH, J = 4,9 Hz); 4,09 (d, 1H, H-1, J = 7,6 Hz); 4,0-3,55 (m, 4H); 3,45 (m, 2H); 3,15-2,9 (m, 4H); 2,1-1,88 (m, 4H); 1,45 (m, 2H); 1,22 (m, 50H, aliphat.); 0,85 (t, 6H, $CH_3$).

## Patentansprüche

1. Verfahren zur Herstellung von Sphingosinderivaten der allgemeinen Formel (I)

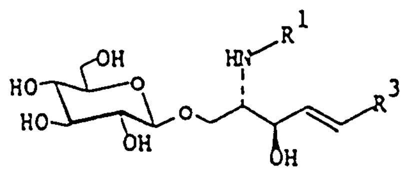

(I)

in welcher Formel $R^1$ den Acylrest einer Fettsäure mit 14 bis 24 Kohlenstoffatomen oder die entsprechenden Acylreste mit einer Hydroxylgruppe in α-Stellung oder mit einer oder zwei Doppelbindungen in cis-Konfiguration und $R^3$ einen aliphatischen Rest mit 13 bis 19 Kohlenstoffatomen, wovon mindestens 13 in gerader Kette und gegebenenfalls höchstens 4 als seitliche Methylgruppen vorliegen, welcher Rest bis zu drei Doppelbindungen von cis- oder trans-Konfiguration oder bis zu drei Dreifachbindungen beinhalten kann, bedeutet, dadurch gekennzeichnet, dass man D-Galactose mit einem niederen aliphatischen Keton oder einem aromatischen Aldehyd der Formel R-CO-R', in welcher R und R' je einen niederen Alkylrest bedeuten bzw. eines von R und R' das Wasserstoffatom und das andere einen aromatischen Rest bedeutet, zu einer in den 4- und 6-Stellungen geschützten D-Galactose der Formel (II) umsetzt, diese Verbindung mit einem vicinale Diole spaltenden Oxydationsmittel zur entsprechenden, in den 2- und 4-Stellungen geschützten D-Threose der Formel (III) aufspaltet, die geschützte D-Threose mit einem $R^3$-$CH_2$-Phosphonat oder einem $R^3$-$CH_2$-Triphenylphosphoniumhalogenid, in welchen $R^3$ die obige Bedeutung besitzt, in Gegenwart einer Base bzw. einer Base und eines Salzes zu einer Verbindung der Formel (IV) umsetzt, in dieser Verbindung die freie Hydroxylgruppe durch Aktivierung in eine Azidogruppe überführt, die erhaltene Azidoverbindung der Formel (V) von der Schutzgruppe der Hydroxylgruppen in den 1- und 3-Stellungen der aliphatischen Kette unter Bildung einer 2-Azido-1,3-dihydroxyverbindung der Formel (VI) befreit, letztere mit einem organischen Reagens, das mit einer primären Hydroxylgruppe selektiv zu reagieren vermag, unter Bildung einer Verbindung der Formel (VIII), in welcher R'' eine Hydroxylschutzgruppe bedeutet, umsetzt, in der Verbindung der Formel (VIII) die sekundäre Hydroxylgruppe mit einer Schutzgruppe R''' blockiert, von der erhaltenen Verbindung der Formel (IX) die Hydroxylschutzgruppe R'' unter Bildung einer Verbindung der Formel (X) abspaltet und entweder die zuvor erhaltene Verbindung der Formel (VI) oder aber die Verbindung der Formel (X) mit dem O-Trifluor- oder O-Trichlor-acetimidat oder dem 1-Halogenderivat einer D-Glucose, deren Hydroxylgruppen in den 2-, 3-, 4- und 6-Stellungen durch Acylreste Ac geschützt sind, zu einer Verbindung der Formel (VII) bzw. (XI) glykosidiert, von der erhaltenen Verbindung die Acylgruppen Ac bzw. die Acylgruppen Ac und die Schutzgruppe R''' unter Bildung derselben Verbindung der Formel (XII) abspaltet, in dieser die Azidogruppe in eine primäre Aminogruppe überführt und die erhaltene Verbindung der Formel (XIII) einer N-Acylierung mit einer Fettsäure der Formel $R^1$-OH unterwirft.

2. Verfahren nach Anspruch 1, dadurch gekennzeichnet, dass als Keton oder Aldehyd der Formel R-CO-R' Aceton, Ethylmethylketon oder Diethylketon beziehungsweise Benzaldehyd oder ein am Phenylring substituierter Benzaldehyd verwendet wird.

3. Verfahren nach Anspruch 1, dadurch gekennzeichnet, dass als Oxydationsmittel ein Alkalimetallperjodat oder Bleitetraacetat verwendet und die Oxydation der Verbindung der Formel (II) bei einem pH-Wert von etwa 7 oder 8 bei Raumtemperatur durchgeführt wird.

4. Verfahren nach Anspruch 1, dadurch gekennzeichnet, dass die Umsetzung der geschützten D-Threose der Formel (III) mit dem $R^3$-$CH_2$-Phosphonat oder dem $R^3$-$CH_2$-Triphenylphosphoniumhalogenid in Gegenwart von Phenyllithium, Lithiumethylat, Natriumamid, Natriummethylat oder Natriumcarbonat in einem wasserfreien Kohlenwasserstoff oder Ether unter Stickstoffatmosphäre bei tiefen Temperaturen und bei Verwendung eines $R^3$-$CH_2$-Phosphoniumhalogenids unter Zusatz eines Salzes durchgeführt wird.

5. Verfahren nach Anspruch 1, dadurch gekennzeichnet, dass die Ueberführung der freien Hydroxylgruppe der Verbindung der Formel (IV) in eine Azidogruppe durch O-Trifluormethansulfonierung, Methansulfonierung oder p-Toluolsulfonierung und anschliessende Umsetzung des O-Sulfonylderivates mit einem Alkalimetallazid durchgeführt wird.

6. Verfahren nach Anspruch 1, dadurch gekennzeichnet, dass die Abspaltung der Schutzgruppe R-CO-R' von der Verbindung der Formel (V) bzw. der Schutzgruppe R'' von der Verbindung der Formel (IX) durch saure Hydrolyse durchgeführt wird.

7. Verfahren nach Anspruch 1, dadurch gekennzeichnet, dass als Hydroxylschutzgruppe R'' eine räumlich grosse Gruppe wie die Triphenylmethyl-, Monomethoxytriphenylmethyl-, tert.Butyl-, Trichloracetyl-, Trimethyl-, tert.Butyldimethylsilyl- oder tert.Butyldiphenylsilylgruppe verwendet wird.

8. Verfahren nach Anspruch 1, dadurch gekennzeichnet, dass als Schutzgruppe R''' der Acylrest einer aliphatischen oder aromatischen Carbonsäure oder eine tert.Butoxycarbonylgruppe, vorzugsweise der Acylrest der Benzoesäure oder einer substituierten Benzoesäure oder der Pivalinsäure, verwendet wird.

9. Verfahren nach Anspruch 1, dadurch gekennzeichnet, dass die Glykosidierung der Verbindung der Formel (VI) bzw. (X) mit besagtem O-Trifluor- oder O-Trichlor-acetimidat in Gegenwart eines Lewis-Säure-Katalysators und in einem wasserfreien Kohlenwasserstoff oder halogenierten Kohlenwasserstoff, jene mit besagtem 1-Halogenderivat in Gegenwart eines säurebindenden Mittels oder eines Schwermetallsalzes durchgeführt wird.

10. Verfahren nach Anspruch 1, dadurch gekennzeichnet, dass die Acylgruppen Ac und die Schutzgruppe R''' von der Verbindung der Formel (VII) bzw. (XI) durch basische Katalyse abgespalten werden.

11. Verfahren nach Anspruch 1, dadurch gekennzeichnet, dass die Ueberführung der Azidogruppe der Verbindung der Formel (XII) in eine primäre Aminogruppe durch Behandlung mit Schwefelwasserstoff in einem Gemisch (1:1) von Wasser und Pyridin oder durch Hydrierung mit Natriumborhydrid oder einem anderen Reduktionsmittel durchgeführt wird.

12. Verfahren nach Anspruch 1, dadurch gekennzeichnet, dass die N-Acylierung der Verbindung der Formel (XIII) mittels der Fettsäure der Formel $R^1$-OH in Gegenwart eines wasserabspaltenden Mittels oder mittels eines aktivierten Esters der Fettsäure oder mittels eines Halogenids derselben in Gegenwart einer anorganischen Base oder einer tertiären organischen Base durchgeführt wird.

**Claims**

1. Process for the preparation of sphingosine derivatives of the general formula (I)

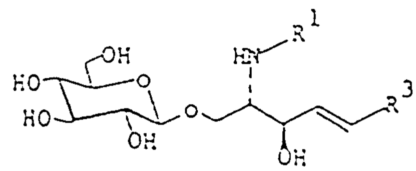

(I)

in which formula R' denotes the acyl radical of a fatty acid with 14 to 24 carbon atoms or the corresponding acyl radicals with a hydroxyl group in the α-position or with one or two double bonds in the cis-configuration and $R^3$ denotes an aliphatic radical with 13 to 19 carbon atoms, at least 13 of which are present in a straight chain and, if appropriate, not more than 4 are present as lateral methyl groups, it being possible for this radical to contain up to three double bonds of cis- or trans-configuration or up to three triple bonds, characterized in that D-galactose is reacted with a lower aliphatic ketone or an aromatic aldehyde of the formula R-CO-R', in which R and R' each denote a lower alkyl radical or one of the radicals R and R' denotes a hydrogen atom and the other denotes an aromatic radical, to give a D-galactose protected in the 4- and 6-positions, of the formula (II), this compound is split with an oxidizing agent which splits vicinal diols, to give the corresponding D-threose protected in the 2- and 4-positions, of the formula (III), the protected D-threose is reacted with an $R^3$-$CH_2$-phosphonate or an $R^3$-$CH_2$-triphenylphosphonium halide, in which $R^3$ has the above meaning, in the presence of a base or of a base and a salt to give a compound of the formula (IV), the free hydroxyl group in this compound is converted into an azido group by activation, the resulting azido compound of the formula (V) is freed from the protective group on the hydroxyl groups in the 1- and 3-position of the aliphatic chain to form a 2-azido-1,3-dihydroxy compound of the formula (VI), the latter is reacted with an organic reagent which is capable of reacting selectively with a primary hydroxyl group, to form a compound of the formula (VIII), in which R" denotes a hydroxyl-protective group, the secondary hydroxyl group in the compound of the formula (VIII) is blocked with a protective group R"', the hydroxyl-protective group R" is split off from the resulting compound of the formula (IX) to form a compound of the formula (X) and either the compound of the formula (VI) obtained previously or the compound of the formula (X) is glycosidated with the 0-trifluoro- or 0-trichloro-acetimidate or the 1-halogen derivative of a D-glucose, the hydroxyl groups of which in the 2-, 3-, 4- and 6-positions are protected by acyl radicals Ac, to give a compound of the formula (VII) or (XI), the acyl groups Ac or the acyl groups Ac and the protective group R"' are split off from the resulting compound to form the same compound of the formula (XII), the azido group in this is converted into a primary amino group and the resulting compound of the formula (XIII) is subjected to N-acylation with a fatty acid of the formula $R^1$-OH.

2. Process according to Claim 1, characterized in that acetone, ethyl methyl ketone or diethyl ketone, or benzaldehyde or a benzaldehyde substituted on the phenyl ring is used as the aldehyde or ketone of the formula R-CO-R'.

3. Process according to Claim 1, characterized in that an alkali metal periodate or lead tetraacetate is used as the oxidizing agent and the oxidation of the compound of the formula (II) is carried out at a pH of about 7 or 8 at room temperature.

4. Process according to Claim 1, characterized in that the reaction of the protected D-threose of the formula (III) with the $R^3$-$CH_2$-phosphonate or the $R^3$-$CH_2$-triphenylphosphonium halide is carried out in the presence of phenyllithium, lithium ethylate, sodium amide, sodium methylate or sodium carbonate in an anhydrous hydrocarbon or ether under a nitrogen atmosphere at low temperatures and, when using an $R^3$-$CH_2$-phosphonium halide, with the addition of a salt.

5. Process according to Claim 1, characterized in that the conversion of the free hydroxyl group of the compound of the formula (IV) into an azido group is carried out by 0-trifluoromethanesulphonation, methanesulphonation or p-toluenesulphonation and subsequent reaction of the 0-sulphonyl derivative with an alkali metal azide.

6. Process according to Claim 1, characterized in that the protective group R-CO-R' is split off from the compound of the formula (V) or the protective group R'' is split off from the compound of the formula (IX) by acid hydrolysis.

7. Process according to Claim 1, characterized in that a spatially large group, such as the triphenylmethyl, monomethoxytriphenylmethyl, tert.-butyl, trichloroacetyl, trimethyl, tert.-butyldimethylsilyl or tert.-butyldiphenylsilyl group is used as the hydroxyl-protective group R''.

8. Process according to Claim 1, characterized in that the acyl radical of an aliphatic or aromatic carboxylic acid or a tert.-butoxycarbonyl group, preferably the acyl radical of benzoic acid or of a substituted benzoic acid or of pivalic acid, is used as the protective group R'''.

9. Process according to Claim 1, characterized in that the glycosidation of the compound of the formula (VI) or (X) with the said 0-trifluoro- or 0-trichloro-acetimidate is carried out in the presence of a Lewis acid catalyst and in an anhydrous hydrocarbon or halogenated hydrocarbon, and that with the said 1-halogen derivative is carried out in the presence of an acid-binding agent or a heavy metal salt.

10. Process according to Claim 1, characterized in that the acyl groups Ac and the protective group R''' are split off from the compound of the formula (VII) or (XI) by basic catalysis.

11. Process according to Claim 1, characterized in that the azido group of the compound of the formula (XII) is converted into a primary amino group by treatment with hydrogen sulphide in a mixture (1:1) of water and pyridine or by hydrogenation with sodium borohydride or another reducing agent.

12. Process according to Claim 1, characterized in that the N-acylation of the compound of the formula (XIII) is carried out by means of the fatty acid of the formula $R^1$-OH in the presence of a dehydrating agent or by means of an activated ester of the fatty acid or by means of a halide thereof in the presence of an inorganic base or a tertiary organic base.

## Revendications

1. Procédé de préparation de dérivés de sphingosine de formule générale (I)

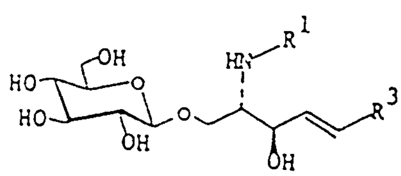

(I)

dans laquelle $R^1$ représente le radical acyle d'un acide gras comportant de 14 à 24 atomes de carbone ou le radical acyle correspondant portant un groupe hydroxyle en position $\alpha$ ou portant une ou deux doubles liaisons de configuration cis et $R^3$ représente un radical aliphatique comportant de 13 à 19 atomes de carbone dont 13 au moins se trouvent en chaîne droite et dont, cas échéant, 4 au plus se trouvent sous forme de groupes méthyliques latéraux, ce radical pouvant comporter jusqu'à trois doubles liaisons de configuration cis ou trans ou jusqu'à trois triples liaisons, caractérisé en ce que l'on fait réagir le D-galactose avec une cétone aliphatique inférieure ou un aldéhyde aromatique de formule R-CO-R', dans laquelle R et R' représentent chacun un radical alcoyle inférieur ou bien l'un de R et R' représente un atome d'hydrogène et l'autre représente un radical aromatique, pour obtenir un D-galactose protégé en les positions 4 et 6, de formule (II), on scinde ce composé au moyen d'un agent d'oxydation capable de scinder les diols vicinaux, pour obtenir un D-thréose protégé en les positions 2 et 4, de formule (III), on fait réagir le D-thréose protégé avec un $R^3$-$CH_2$-phosphonate ou un halogénure de $R^3$-$CH_2$-triphénylphosphonium, dans lesquels $R^3$ a la signification indiquée ci-dessus, en présence d'une base ou d'une base et d'un sel, pour obtenir un composé de formule (IV), on transforme le groupe hydroxyle libre de ce composé, par activation, en un groupe azido, on libère le composé azido

obtenu, de formule (V) des groupes protecteurs des groupes hydroxyle en les positions 1 et 3 de la chaîne aliphatique avec formation d'un composé 2-azido-1,3-dihydroxy de formule (VI), on fait réagir ce dernier avec un réactif organique qui est capable de réagir de manière sélective avec un groupe hydroxyle primaire, avec formation d'un composé de formule (VIII), dans laquelle R'' représente le groupe protecteur d'un hydroxyle, on bloque le groupe hydroxyle secondaire dans le composé de formule (VIII) au moyen d'un groupe protecteur R''', on scinde du composé obtenu de formule (IX) le groupe protecteur de l'hydroxyle R'', avec formation d'un composé de formule (X) et, au moyen du 0-trifluor- ou 0-trichlor-acétimidate ou du dérivé 1-halogéné d'un D-glucose dont les groupes hydroxyle en les positions 2, 3, 4 et 6 sont protégés par des radicaux acyle, on transforme en un composé glucosilé de formule (VII) ou (XI) soit le composé obtenu précédemment de formule (VI), soit au contraire le composé de formule (X), on scinde du composé obtenu les groupes acyle Ac ou les groupes acyle Ac et le groupe protecteur R''' avec formation d'un même composé de formule (XII), on transforme le groupe azido de celui-ci en un groupe amino primaire et on soumet le composé obtenu de formule (XIII) à une N-acylation au moyen d'un acide gras de formule $R^1$-OH.

**2.** Procédé selon la revendication 1, caractérisé en ce que l'on utilise, comme cétone ou aldéhyde de formule R-CO-R', l'acétone, l'éthylméthylcétone ou la diéthylcétone, ou bien le benzaldéhyde ou un benzaldéhyde substitué sur le cycle phénylique.

**3.** Procédé selon la revendication 1, caractérisé en ce que l'on utilise comme agent d'oxydation un periodate de métal alcalin ou le tétraacétate de plomb et que l'oxydation du composé de formule (II) est effectuée à un pH d'environ 7 ou 8, à la température ordinaire.

**4.** Procédé selon la revendication 1, caractérisé en ce que la réaction du D-thréose protégé de formule (III) avec le $R^3$-$CH_2$-phosphonate ou l'halogénure de $R^3$-$CH_2$-triphénylphosphonium est effectuée en présence de phényllithium, de méthylate de lithium, d'amidure de sodium, de méthylate de sodium ou de carbonate de sodium, dans un hydrocarbure ou un éther anhydre, sous atmosphère d'azote, à basses températures et, lorsqu'on emploie un halogénure de $R^3$-$CH_2$-phosphonium, avec addition d'un sel.

**5.** Procédé selon la revendication 1, caractérisé en ce que la transformation du groupe hydroxyle libre du composé de formule (IV) en un groupe azido est effectuée par une O-trifluorméthanesulfonation, méthanesulfonation ou p-toluènesulfonation et réaction subséquente du dérivé O-sulfonyle avec un azidure de métal alcalin.

**6.** Procédé selon la revendication 1, caractérisé en ce que la scission du groupe protecteur R-CO-R' du composé de formule (V) ou du groupe protecteur R'' du composé de formule (IX) est effectuée par hydrolyse acide.

**7.** Procédé selon la revendication 1, caractérisé en ce que l'on emploie, comme groupe protecteur de l'hydroxyle R'', un groupe volumineux tel que le groupe triphénylméthyle, monométhoxytriphénylméthyle, tert.butyle, trichloracétyle, triméthyl-, tert.butyldiméthylsilyle ou tert.butyldiphénylsilyle.

**8.** Procédé selon la revendication 1, caractérisé en ce que l'on emploie comme groupe protecteur R''' le radical acyle d'un acide carbonique aliphatique ou aromatique ou un groupe tert. butoxycarbonyle, de préférence le radical acyle de l'acide benzoïque ou d'un acide benzoïque substitué ou de l'acide pivalique.

**9.** Procédé selon la revendication 1, caractérisé en ce que la transformation du composé de formule (VI) ou (X) en un glucoside est effectuée avec ledit O-trifluor- ou O-trichlor-acétimidate en présence d'un catalyseur acide de Lewis et dans un hydrocarbure ou un hydrocarbure halogéné anhydre, la réaction avec ledit dérivé 1-halogéné, en présence d'un agent de liaison des acides ou d'un sel de métal lourd.

**10.** Procédé selon la revendication 1, caractérisé en ce que l'on scinde les groupes acyle Ac et le groupe protecteur R''' du composé de formule (VII) ou (IX) par catalyse basique.

**11.** Procédé selon la revendication 1, caractérisé en ce que l'on effectue la transformation du groupe azido du composé de formule (XII) en un groupe amino primaire par traitement avec de l'hydrogène sulfuré

dans un mélange d'eau et de pyridine (1 : 1) ou par hydrogénation au moyen de borohydrure de sodium ou d'un autre agent réducteur.

**12.** Procédé selon la revendication 1, caractérisé en ce que l'on effectue la N-acylation du composé de formule (XIII) au moyen de l'acide gras de formule $R^1$-OH en présence d'un agent d'élimination de l'eau ou au moyen d'un ester activé de l'acide gras ou au moyen d'un halogénure de celui-ci en présence d'une base minérale ou d'une base organique tertiaire.

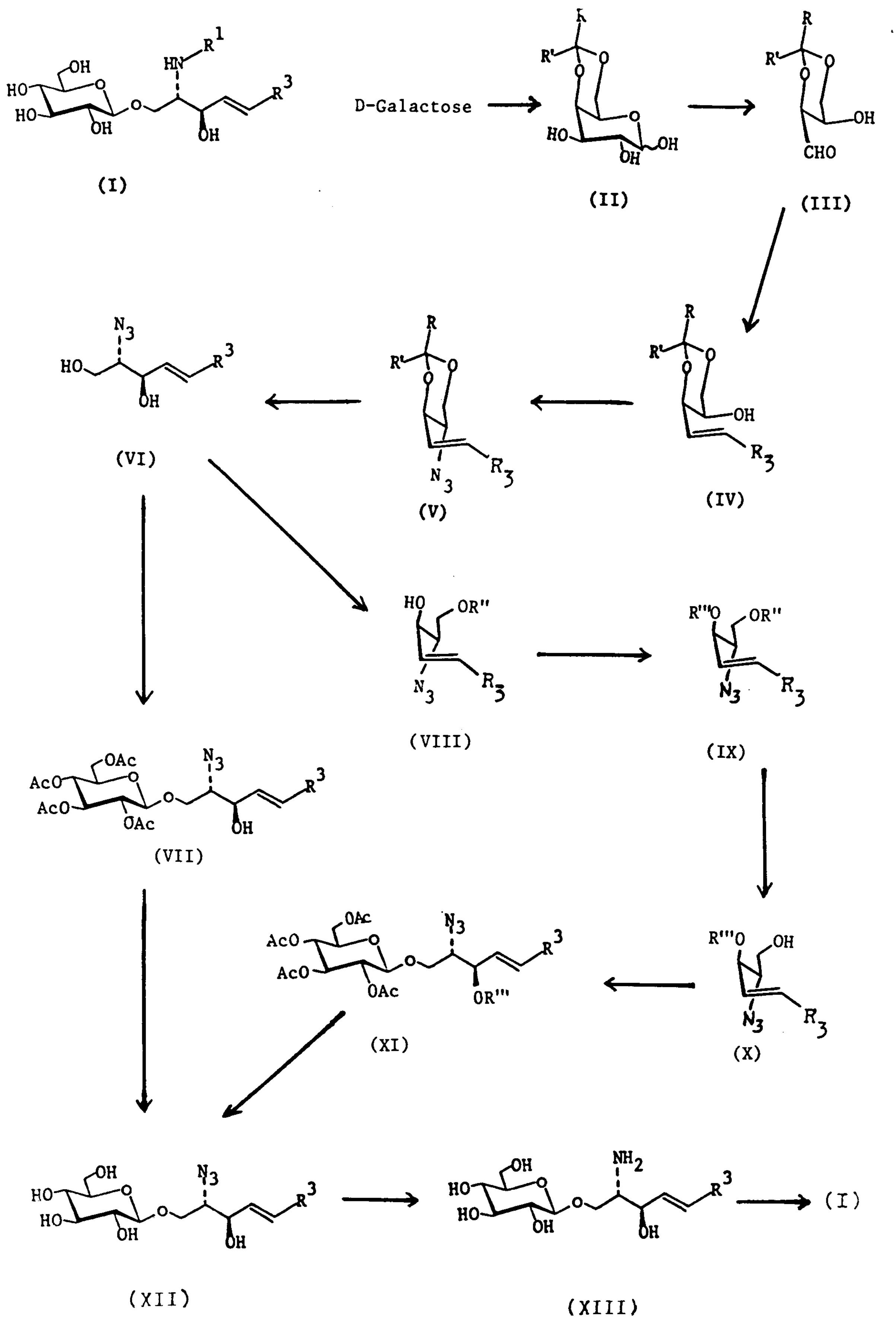


(I) (II) (III) (IV) (V) (VI) (VII) (VIII) (IX) (X) (XI) (XII) (XIII)